# EUROPEAN PATENT APPLICATION

(11) **EP 4 035 599 A1**
(43) Date of publication of application: **03.08.2022**
(21) Application number: 22152802.9
(22) Date of filing: 21.01.2022
(51) Int. Cl.: A61B 5/16, A61B 5/245, A61B 5/00, G16H 50/30

(54) **BRAIN FUNCTION EVALUATION SYSTEM, METHOD, AND COMPUTER-READABLE MEDIUM**

(30) Priority: 29.01.2021 JP 2021014012
(71) Applicant: Ricoh Company, Ltd., Tokyo 143-8555 (JP)
(72) Inventor: Koide, Yasuhisa, Tokyo, 143-8555 (JP); Sakai, Daisuke, Tokyo, 143-8555 (JP)
(74) Representative: Watkin, Timothy Lawrence Harvey

(57) **Abstract**

A brain function evaluation system (1) includes a receiving unit (100a), and a processing unit (100b). The receiving unit (100a) is configured to receive examinee information on a brain function. The processing unit (100b) is configured to calculate an evaluation result by evaluating a brain function based on the examinee information and additional examinee information.

## Description

### BACKGROUND OF THE INVENTION

### 1. Field of the Invention

The present invention relates to a brain function evaluation system, a method, and a computer-readable medium.

### 2. Description of the Related Art

Conventionally, with respect to a brain function, such as a cognitive function, a medical interview and a plurality of kinds of measurement, such as brain's magnetic field measurement, using magnetoencephalograph are performed on a patient, and a doctor makes a diagnosis on a state of the brain function of the patient from an obtained result. After the doctor has made the diagnosis, if treatment is started, the patient periodically receives a medical interview or brain's magnetic field measurement in a hospital and follows a decision made by the doctor on the treatment, such as continuation or change of the treatment. In recent years, a diagnosis assist system that assists the doctor in making a diagnosis has been widespread; however, functions provided by the diagnosis assist system are limited to only a function to display a measurement result or the like in an easily viewable manner, a function to generate a report, and the like.

Japanese Patent No. 6475132 discloses a report generation system that is able to display treatment and medication information and biological signals in chronological order in a single report.

However, a plurality of methods are provided as a method of measuring a brain function, and the conventional diagnosis assist system is not able to evaluate the brain function while adding information obtained through measurement, which is a problem.

An object of the present invention is to provide a brain function evaluation system, a method, and a computer readable medium capable of evaluating a brain function as the occasion demands, on the basis of information on the brain function obtained from an examinee and additional examinee information.

### SUMMARY OF THE INVENTION

According to an aspect of the present invention, a brain function evaluation system includes a receiving unit, and a processing unit. The receiving unit is configured to receive examinee information on a brain function. The processing unit is configured to calculate an evaluation result by evaluating a brain function based on the examinee information and additional examinee information.

According to an aspect of the present invention, it is possible to evaluate a brain function on an as the occasion demands, on the basis of information on a brain function obtained from an examinee and additional examinee information.

### BRIEF DESCRIPTION OF THE DRAWINGS

FIG. 1 is a diagram illustrating an example of an entire configuration of a brain function evaluation system according to an embodiment;
FIG. 2 is a diagram illustrating an example of a hardware configuration of a web server;
FIG. 3 is a diagram illustrating an example of a hardware configuration of a client personal computer (PC);
FIG. 4 is a diagram illustrating an example of a functional block configuration of the brain function evaluation system;
FIG. 5 is a diagram illustrating an example of an entire sequence in the brain function evaluation system;
FIG. 6 is a diagram illustrating an example of a magnetoencephalography data registration screen that is displayed on a UI screen;
FIG. 7 is a diagram illustrating an example of an input screen for questions and answers for a medical interview;
FIG. 8 is a diagram illustrating an example of an input screen for inputting a result of a psychological test;
FIG. 9 is a diagram illustrating an example of a flow of determining a brain function risk by a brain function risk calculation unit;
FIGS. 10A and 10B are diagrams illustrating an example of reference data (determination criteria) for determining the brain function risk;
FIG. 11 is a diagram illustrating an example of a flow of determining a cognitive function risk by a cognitive function calculation unit;
FIGS. 12A and 12B are diagrams illustrating an example of reference data for determining the cognitive function risk;
FIG. 13 is a diagram illustrating an example of a flow of determining a lifestyle risk by a lifestyle risk calculation unit;
FIGS. 14A and 14B are diagrams illustrating an example of reference data for determining the lifestyle risk;
FIG. 15 is a diagram illustrating an example of a comprehensive evaluation flow implemented by the comprehensive evaluation calculation unit;
FIG. 16 is a diagram illustrating an example of a flow of a subroutine of the comprehensive evaluation flow;
FIG. 17 is a diagram illustrating an example of reference data used for risk classification to perform a comprehensive evaluation;
FIG. 18 is a diagram illustrating an example of a configuration of a report that is generated by a report generation unit;
FIG. 19 is a diagram illustrating an example of comment data for each of categories of a comprehensive evaluation, which is used in the report; and
FIG. 20 is a diagram illustrating a display example in which changes in the comprehensive evaluation are displayed in chronological order on the UI screen.

The accompanying drawings are intended to depict exemplary embodiments of the present invention and should not be interpreted to limit the scope thereof. Identical or similar reference numerals designate identical or similar components throughout the various drawings.

### DESCRIPTION OF THE EMBODIMENTS

The terminology used herein is for the purpose of describing particular embodiments only and is not intended to be limiting of the present invention.

As used herein, the singular forms "a", "an" and "the" are intended to include the plural forms as well, unless the context clearly indicates otherwise.

In describing preferred embodiments illustrated in the drawings, specific terminology may be employed for the sake of clarity. However, the disclosure of this patent specification is not intended to be limited to the specific terminology so selected, and it is to be understood that each specific element includes all technical equivalents that have the same function, operate in a similar manner, and achieve a similar result.

An embodiment of the present invention will be described in detail below with reference to the drawings. Embodiments of a brain function evaluation system, a method, and a computer readable medium will be described in detail below with reference to the accompanying drawings.

### First Embodiment

A brain function evaluation system according to the first embodiment provides a comprehensive evaluation function to comprehensively evaluate a brain function on the basis of data (examinee information) on a brain function of an examinee. As one example, the examinee information is information for evaluating each of a brain function risk, a cognitive function risk, and a lifestyle risk, and the comprehensive evaluation function capable of calculating an evaluation result that comprehensively evaluates the brain function even from one of the pieces of information as described above. Further, it makes it possible to, if additional examinee information, such as remaining examinee information that is not yet registered or update information on the already-registered examinee information, is present, calculate an evaluation result by comprehensively re-evaluating the brain function while reflecting the added or updated information, and store or output the calculated evaluation result in each case. Meanwhile, a timing of calculating the evaluation result of re-evaluation is as the occasion demands. For example, the evaluation result of re-evaluation is calculated at a predetermined moment, such as when a user operates a button for requesting calculation of the evaluation result. In the following, calculation of the evaluation result will be described as an "evaluation" for the sake of convenience. Here, the "brain function risk" is an index that classifies a risk of reduction in the brain function by index values (for example, levels A, B, and C) on the basis of magnetoencephalography data, a biological signal, or the like of an examinee. The "cognitive function risk" is an index that classifies a risk in a cognitive function by index values (for example, levels A, B, and C) on the basis of a result of a psychological test, a medical interview, or the like performed on the examinee. The "lifestyle risk" is an index that classifies a risk of disease or the like caused by a lifestyle by index values (for example, levels A, B, and C) on the basis of a self-enumeration on the lifestyle of the examinee. A "brain function" indicated by a comprehensive brain function evaluation is different from a "brain function" in the "brain function risk", indicates a new comprehensive evaluation on the brain, which is classified based on factors including each of the risks, and may be used as a "brain function evaluation" in a "brain function checkup", for example.

### Overall system

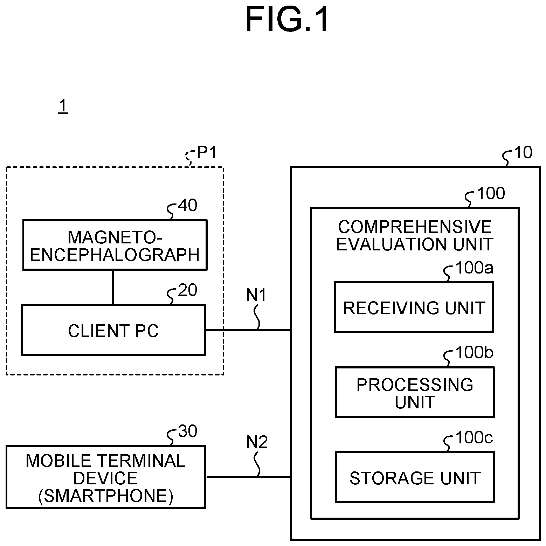

FIG. 1 is a diagram illustrating an example of an entire configuration of the brain function evaluation system according to the embodiment. A brain function evaluation system 1 illustrated in FIG. 1 may be embodied in the form of a client-server system, for example. A mode in which a web server system is adopted will be described below as one example.

In the configuration illustrated in FIG. 1, a web server 10 is an information processing apparatus having a computer configuration. The web server 10 includes a comprehensive evaluation unit 100 that comprehensively evaluates a brain function of a patient.

The web server 10 comprehensively evaluates the brain function of the patient on the basis of patient information that is received from a client PC and a mobile terminal device 30, and transmits an evaluation result to the client PC and the mobile terminal device 30. Specifically, the comprehensive evaluation unit 100 of the web server 10 includes a receiving unit 100a, a processing unit 100b, and a storage unit 100c. The receiving unit 100a receives various kinds of information. In this example, the receiving unit 100a receives the patient information that is examinee information for determining a risk in the brain function. The processing unit 100b performs various kinds of processing on the basis of the received information. In this example, the processing unit 100b comprehensively evaluates the brain function, that is, calculates an evaluation result, on the basis of the patient information received by the receiving unit 100a. A calculation result is stored in the storage unit 100c as the occasion demands, or output to an external apparatus that has issued a request. Meanwhile, the storage unit 100c is arranged in the web server 10 in this configuration example, but the storage unit 100c may be arranged in an external storage or the like.

A client personal computer (PC) 20 and the mobile terminal device 30 access the web server 10 via a network.

The client PC 20 is a medical staff terminal in a hospital P1 (or in a medical examination center or the like). In the example illustrated in FIG. 1, a PC that is connected to a measurement device, such as a magnetoencephalograph 40 is illustrated, but it may be possible to adopt a PC that is able to acquire data of the measurement device and that is installed in a medical examination or the like. The client PC 20 is connectable to a first network N1 and transmits the patient information to the web server 10 via the first network N1. The first network N1 is a certain network, such as a local area network (LAN) or the Internet, which connects the client PC 20 and the web server 10.

The mobile terminal device 30 is a portable terminal device, such as a smartphone or a tablet terminal device. The mobile terminal device 30 is connectable to the second network N2 and transmits the patient information to the web server 10 via the second network N2. The second network N2 is a certain network, such as Wi-Fi (registered trademark), a public network, or the Internet, which connects the mobile terminal device 30 and the web server 10.

Meanwhile, the mobile terminal device 30 corresponds to an "examinee terminal". The mobile terminal device 30 includes a smartphone that is widely used, and, for example, even an external staff who assists treatment on the patient, a family member of the patient, or the patient him or herself is able to use a part or all of functions of the web server 10 through a public network from outside, such as home or a facility, as long as an access to the web server 10 is authenticated.

The web server 10 may be implemented by a single information processing apparatus or may be implemented by a combination of a plurality of information processing apparatuses. Further, a part or all of the functions of the comprehensive evaluation unit 100 for evaluating the brain function may be provided by cloud computing. Furthermore, a part of the functions provided in the web server 10 (a part of the functions for comprehensively evaluating the brain function) may be provided on the client PC 20 or the mobile terminal device 30.

Moreover, the medical staff terminal and the n terminal are not limited to the client PC 20 and the mobile terminal device 30. The apparatus configurations and the numbers of the medical staff terminals and the examinee terminals may be determined arbitrarily.

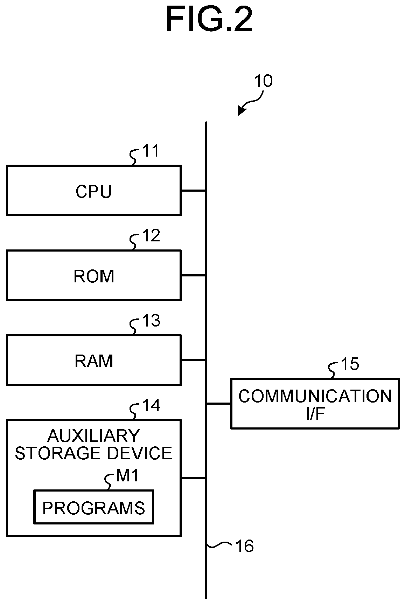

FIG. 2 is a diagram illustrating an example of a hardware configuration of the web server 10. As illustrated in FIG. 2, the web server 10 includes a central processing unit (CPU) 11, a read only memory (ROM) 12, a random access memory (RAM) 13, an auxiliary storage device 14, and a communication interface (I/F) 15. All of the units are connected to one another via a bus 16 or the like.

The CPU 11 is a central arithmetic processor and controls the entire web server 10. The ROM 12 is a memory for storing a fixed program, such as BIOS. The RAM 13 is a memory that is used as a work area or the like when the CPU 11 performs a process.

The auxiliary storage device 14 is a hard disk drive (HDD), a solid state drive (SSD), or the like. The auxiliary storage device 14 stores therein various programs, data, or the like of the web server 10. The various programs include an operating system (OS) of the web server 10, a program for evaluating the brain function, and the like.

The communication I/F 15 is a network interface for performing communication with the client PC 20 and the mobile terminal device 30.

The CPU 11 of the web server 10 reads various programs M1 from the auxiliary storage device 14 onto the RAM 13 and executes the programs M1 to implement, as a functional unit, each of the units (the receiving unit 100a, the processing unit 100b, and the storage unit 100c) for comprehensively evaluating the brain function.

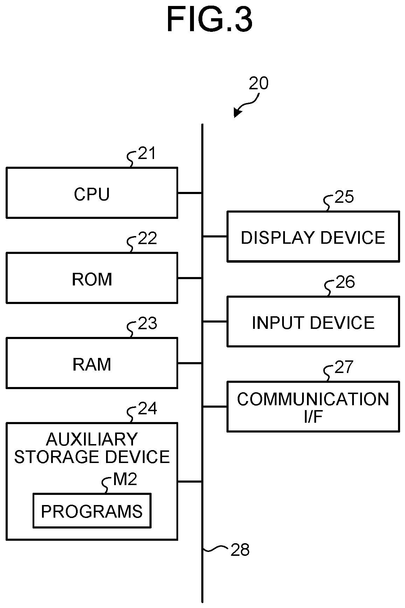

FIG. 3 is a diagram illustrating an example of a hardware configuration of the client PC 20. As illustrated in FIG. 3, the client PC 20 includes a CPU 21, a ROM 22, a RAM 23, an auxiliary storage device 24, a display device 25, an input device 26, and a communication I/F 27. All of the units are connected to one another via a bus 28 or the like.

The CPU 21 is a central arithmetic processor and controls the entire client PC 20. The ROM 22 is a memory for storing a fixed program, such as BIOS. The RAM 23 is a memory that is used as a work area or the like when the CPU 21 performs a process.

The auxiliary storage device 24 is an HDD, an SSD, or the like. The auxiliary storage device 24 stores therein various programs, data, or the like of the client PC 20. The various programs include an OS of the client PC 20 and programs M2, such as a web viewer.

The display device 25 is a liquid crystal display (LCD), an organic electroluminescence (EL) display, or the like. The input device 26 is an input device, such as a keyboard or a mouse. It may be possible to arrange a touch panel as the input device 26. The communication I/F 27 is a network interface for the first network N1.

The CPU 21 of the client PC 20 reads the various programs M2 from the auxiliary storage device 24 onto the RAM 23 and executes the programs M2 to implement a client function (including the web viewer), such as a user interface (UI) unit or a communication unit, that uses the functions of the web server 10.

The hardware configuration of the mobile terminal device 30 can be implemented by the same computer configuration of the client PC 20. Meanwhile, a communication I/F of the mobile terminal device 30 is a network interface for a second network. Further, the CPU of the mobile terminal device 30 reads various programs from an auxiliary storage device onto a RAM and executes the various programs to implement a UI unit, a communication unit, or the like that uses the functions of the web server 10. Other configurations are the same as described in the hardware configuration of the client PC 20, and therefore, illustration and explanation thereof will be omitted.

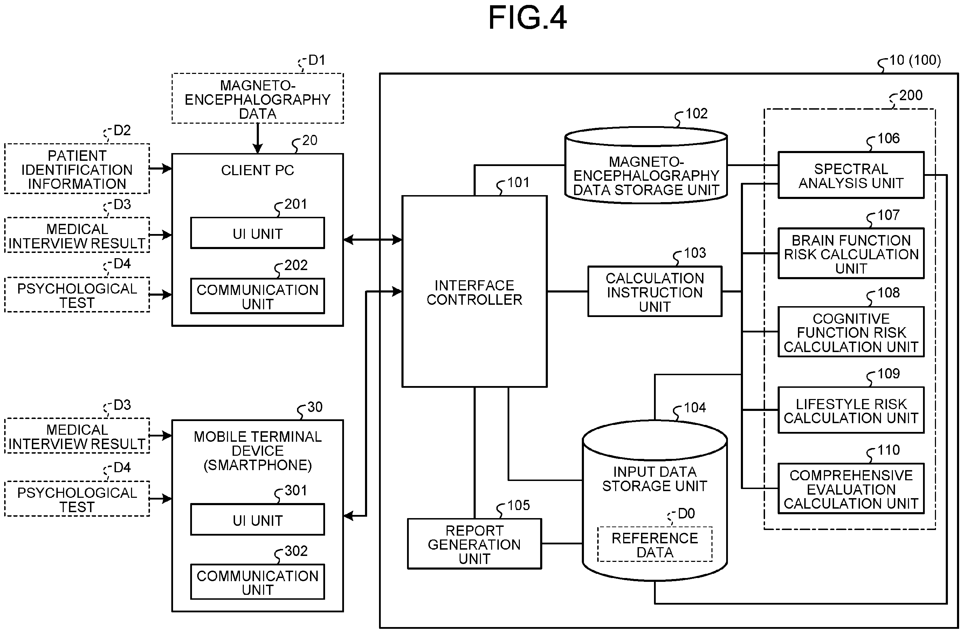

FIG. 4 is a diagram illustrating an example of a functional block configuration of the brain function evaluation system 1. In the web server 10, details of the functional units (the receiving unit 100a, the processing unit 100b, and the storage unit 100c) that comprehensively evaluate the brain function are illustrated. The web server 10 includes an interface controller 101, a magnetoencephalography data storage unit 102, a calculation instruction unit 103, an input data storage unit 104, a report generation unit 105, a spectral analysis unit 106, a brain function risk calculation unit 107, a cognitive function risk calculation unit 108, a lifestyle risk calculation unit 109, and a comprehensive evaluation calculation unit 110.

Here, the interface controller 101 corresponds to the receiving unit 100a (see FIG. 1). The magnetoencephalography data storage unit 102 and the input data storage unit 104 correspond to the storage unit 100c (see FIG. 1). The interface controller 101, the calculation instruction unit 103, the report generation unit 105, the spectral analysis unit 106, the brain function risk calculation unit 107, the cognitive function risk calculation unit 108, the lifestyle risk calculation unit 109, and the comprehensive evaluation calculation unit 110 correspond to the processing unit 100b (see FIG. 1).

The interface controller 101 controls functions to evaluate the brain function, on the basis of accesses from the client PC 20 and the mobile terminal device 30. For example, the interface controller 101 receives a request or reception data from the client PC 20 or the mobile terminal device 30, registers the reception data obtained from the client PC 20 or the mobile terminal device 30, gives various execution instructions on an evaluation of the brain function, or transmits screen information to the client PC 20 or the mobile terminal device 30 that has issued a request.

The magnetoencephalography data storage unit 102 is a storage unit for storing magnetoencephalography data D1 transmitted from the client PC 20. The magnetoencephalography data D1 is a file of magnetoencephalography data obtained by a magnetoencephalograph. The file may include a biological signal, such as a heart rate or a pulse.

The calculation instruction unit 103 instructs a calculation processing unit 200 to perform calculation.

The input data storage unit 104 is a storage unit for storing various kinds of data. For example, the input data storage unit 104 stores therein patient data that is transmitted patient information. The patient data includes input data, such as patient identification information D2 indicating a name, an age, a gender, or the like of the patient, a medical interview result D3 (information listened from the patient or the like) of the patient, a psychological test D4 (a result of a psychological test) performed on the patient, or a calculation result obtained by the calculation processing unit 200. As for the input data, such as the patient identification information D2, the medical interview result D3 of the patient, or the psychological test D4 performed on the patient, the input data transmitted from the client PC 20 or the mobile terminal device 30 is received and stored. Further, the input data storage unit 104 may store therein reference data D0 (including determination reference data) in a modifiable manner. The reference data D0 is different for each of a brain function risk, a cognitive function risk, and a lifestyle risk, and is used by the calculation processing unit 200 to determine each of the risks. If it is acceptable that the reference data D0 is fixed data, it is allowable to hold the data in the calculation processing unit 200.

The report generation unit 105 generates a report for each of patients on the basis of various kinds of data on the patients stored in the input data storage unit 104. For example, if a patient registers a psychological test every day, a comprehensive evaluation result is generated every day, so that time-series information (a broken line graph or the like) on the comprehensive evaluation results is included in the report.

As one example, the calculation processing unit 200 includes the spectral analysis unit 106, the brain function risk calculation unit 107, the cognitive function risk calculation unit 108, the lifestyle risk calculation unit 109, and the comprehensive evaluation calculation unit 110. As the reference data D0, pieces of reference data corresponding to the brain function risk calculation unit 107, the cognitive function risk calculation unit 108, the lifestyle risk calculation unit 109, and the comprehensive evaluation calculation unit 110 are prepared. In the following description, as one example, it is assumed that the reference data D0 is stored in the input data storage unit 104.

If the interface controller 101 receives the magnetoencephalography data D1 from the client PC 20, the spectral analysis unit 106 performs a spectral analysis on the magnetoencephalography data D1. For example, when receiving the magnetoencephalography data D1 from the client PC 20, the interface controller 101 stores the magnetoencephalography data D1 in the magnetoencephalography data storage unit 102, and instructs the calculation instruction unit 103 to perform a spectral analysis on the magnetoencephalography data D1. Accordingly, the spectral analysis unit 106 performs the spectral analysis on the magnetoencephalography data D1. In the spectral analysis, a power spectral value for evaluating a risk of reduction in the brain function is calculated from a biological signal or the like, and an analysis result is stored in the input data storage unit 104.

The brain function risk calculation unit 107 classifies a brain function risk with use of the patient data stored in the input data storage unit 104 and on the basis of the reference data for the brain function risk, and stores a result in the input data storage unit 104.

The cognitive function risk calculation unit 108 classifies a cognitive function risk with use of the patient data stored in the input data storage unit 104 and on the basis of the reference data for the cognitive function risk, and stores a result in the input data storage unit 104.

The lifestyle risk calculation unit 109 classifies a lifestyle risk with use of the patient data stored in the input data storage unit 104 and on the basis of the reference data for the lifestyle risk, and stores a result in the input data storage unit 104.

The comprehensive evaluation calculation unit 110 implements a comprehensive evaluation flow to perform a comprehensive evaluation, and stores a result of the comprehensive evaluation in the input data storage unit 104. For example, if a request for a comprehensive evaluation on the brain function is issued by the client PC 20 or the mobile terminal device 30, the comprehensive evaluation calculation unit 110 performs the comprehensive evaluation while depending on each of the results obtained by the brain function risk calculation unit 107, the cognitive function risk calculation unit 108, and the lifestyle risk calculation unit 109 (corresponding to an "evaluation result", and will be referred to as a "determination result" below), and stores a comprehensive evaluation result in the input data storage unit 104. Meanwhile, while details will be described later, if it is possible to evaluate at least a part of the brain function risk, the cognitive function risk, and the lifestyle risk, the comprehensive evaluation flow is implemented and the comprehensive evaluation is performed. In the example described in the present embodiment, it is sufficient to obtain the results of at least two of the risks. Further, it is more preferable to obtain the results of the three risks because it is possible to obtain a comprehensive evaluation result with high accuracy.

The client PC 20 includes a UI unit 201, such as a web viewer, and a communication unit 202. The UI unit 201 analyzes the screen information that is transmitted from the interface controller 101 of the web server 10 to the communication unit 202, constructs a UI screen, and displays the UI screen. Further, the UI unit 201 transmits input data received through the UI screen, the acquired magnetoencephalography data D1, or an execution command, as request data, from the communication unit 202 to the interface controller 101. For example, the UI unit 201 acquires the magnetoencephalography data D1 measured by the magnetoencephalograph 40 (see FIG. 1) from a designated storage destination. If the client PC 20 is an apparatus that is able to control the magnetoencephalograph 40, the UI unit 201 may display an operation screen of the magnetoencephalograph 40, perform setting or measurement in the magnetoencephalograph 40, and acquire the magnetoencephalography data D1. Further, the UI unit 201 displays an input screen by the display device 25 (see FIG. 2) and receives input of various kinds of data through the screen. For example, the UI unit 201 receives input of the patient identification information D2, the medical interview result D3 of the patient, the psychological test D4 performed on the patient, and the like from the input device 26 (see FIG. 2).

If an instruction on transmission is issued from the UI screen to the web server 10, the UI unit 201 transmits request information to the web server 10 via the communication unit 202. For example, if an instruction on transmission of the patient identification information is issued from the UI screen, the UI unit 201 transmits request information indicating a registration request and the patient identification information D2 to the web server 10 via the communication unit 202. Further, if an instruction on transmission of the magnetoencephalography data is issued from the UI screen, the UI unit 201 transmits request information indicating a registration request and the magnetoencephalography data D1 to the web server 10 via the communication unit 202. Furthermore, if an instruction on transmission of a medical interview result or a psychological test is issued from the UI screen, the UI unit 201 transmits request information indicating a registration request and the input data, such as the medical interview result D3 or the psychological test D4, to the web server 10 via the communication unit 202. Moreover, if an instruction on transmission of a comprehensive evaluation on the brain function from the UI screen, the UI unit 201 transmits request information indicating an acquisition request to the web server 10 via the communication unit 202.

Furthermore, the UI unit 201 may display the UI screen and register or update the reference data D0 with respect to the web server 10.

The mobile terminal device 30 includes a UI unit 301 and a communication unit 302 similarly to the client PC 20. In general, the mobile terminal device 30 is not able to acquire the magnetoencephalography data D1, and therefore, the UI unit 301 may be mainly limited to a function to register the medical interview result D3, the psychological test D4, and the like, a function to update a comprehensive evaluation on the brain function and display a history, and the like.

### Sequence of entire system

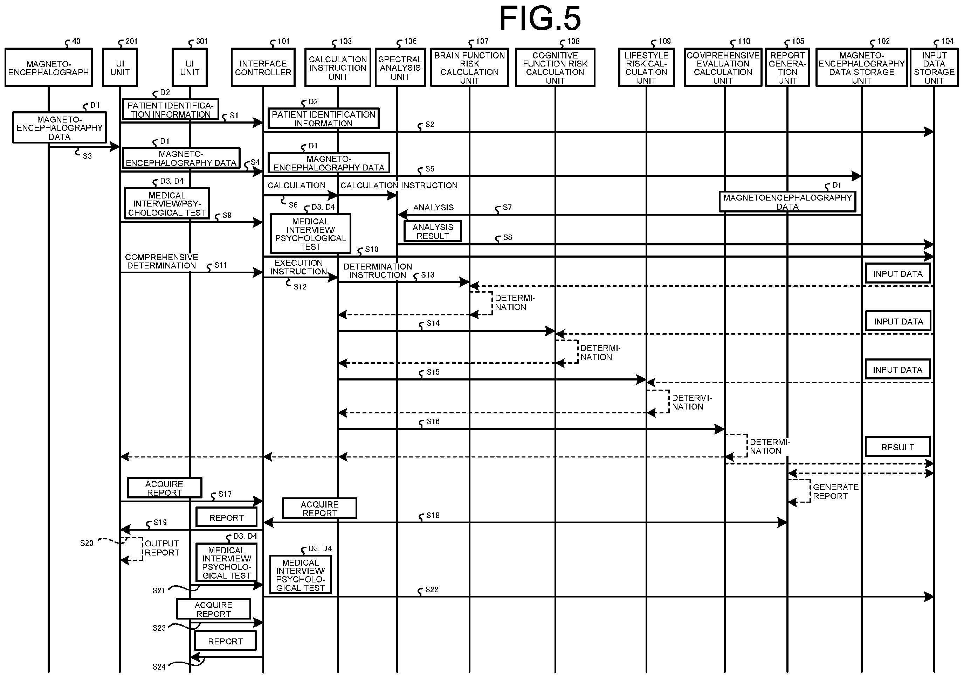

FIG. 5 is a diagram illustrating an example of an entire sequence in the brain function evaluation system 1. While not specifically described below, it is assumed that the client PC 20 and the web server 10 perform communication via the first network N1. Further, it is assumed that the mobile terminal device 30 and the web server 10 perform communication via the second network N2.

First, a medical examination representative in a hospital inputs the patient identification information D2 on a patient who has visited the hospital, by using the UI screen that is acquired by the client PC 20 from the web server 10, and transmits input data to the web server 10 (the interface controller 101) (S1). Here, the medical examination representative is assumed as a primary doctor or a medical staff who performs a medical examination on the patient.

The interface controller 101 registers the patient identification information D2 transmitted from the client PC 20 in the input data storage unit 104 (S2). The registered patient identification information D2 is managed by information unique to the patient. The information unique to the patient is used by issuing a patient ID or the like when the patient has the medical examination. The patient ID may be used as authentication information at a later time. The interface controller 101 uses the information unique to the patient, and even at a later time, if data related to the patient is present, the interface controller 101 stores the data in the input data storage unit 104 in association with the information unique to the patient. In other words, data related to the patient is associated with the patient ID for each of patients in the input data storage unit 104.

If registration to the web server 10 is completed, the medical examination representative operates the UI screen, acquires, as a file, measurement data (that is, the magnetoencephalography data D1) on the patient measured by the magnetoencephalograph from a storage destination in which the data is stored by the magnetoencephalograph, and stores the file of the magnetoencephalography data D1 on the patient in the storage unit of the client PC 20 (S3).

Subsequently, the medical examination representative operates the UI screen and transmits the magnetoencephalography data D1 on the patient stored in the storage unit to the web server 10 (the interface controller 101) (S4). The interface controller 101 stores the magnetoencephalography data D1 on the patient transmitted from the client PC 20 in the magnetoencephalography data storage unit 102 (S5), and instructs the spectral analysis unit 106 to perform a spectral analysis on the magnetoencephalography data D1 on the patient through the calculation instruction unit 103 (S6).

The spectral analysis unit 106 acquires the magnetoencephalography data D1, performs the spectral analysis on the magnetoencephalography data D1 (S7), and stores an analysis result in the input data storage unit 104 (S8).

If registration to the web server 10 is completed, the medical examination representative operates the UI screen, inputs the medical interview result on the patient or a result of the psychological test, and transmits the input data (the medical interview result D3 or the psychological test D4) to the web server 10 (the interface controller 101) (S9). Meanwhile, if any of the medical interview and the psychological test is not performed, it may be possible to input a remaining medical interview result or a remaining psychological test at a later time, and then transmit the input data to the web server 10.

The interface controller 101 registers the input data on the patient transmitted from the client PC 20 in the input data storage unit 104 in association with the patient ID of the patient (S10).

After the registration, the medical examination representative operates the UI screen and requests the web server 10 (the interface controller 101) to determine a brain health condition of the patient, that is, to perform a comprehensive evaluation on the brain function (S11).

Upon receiving the request for determination on the brain health condition of the patient from the client PC 20, the interface controller 101 causes the calculation instruction unit 103 to perform a process of determining the brain health condition of the patient (S12).

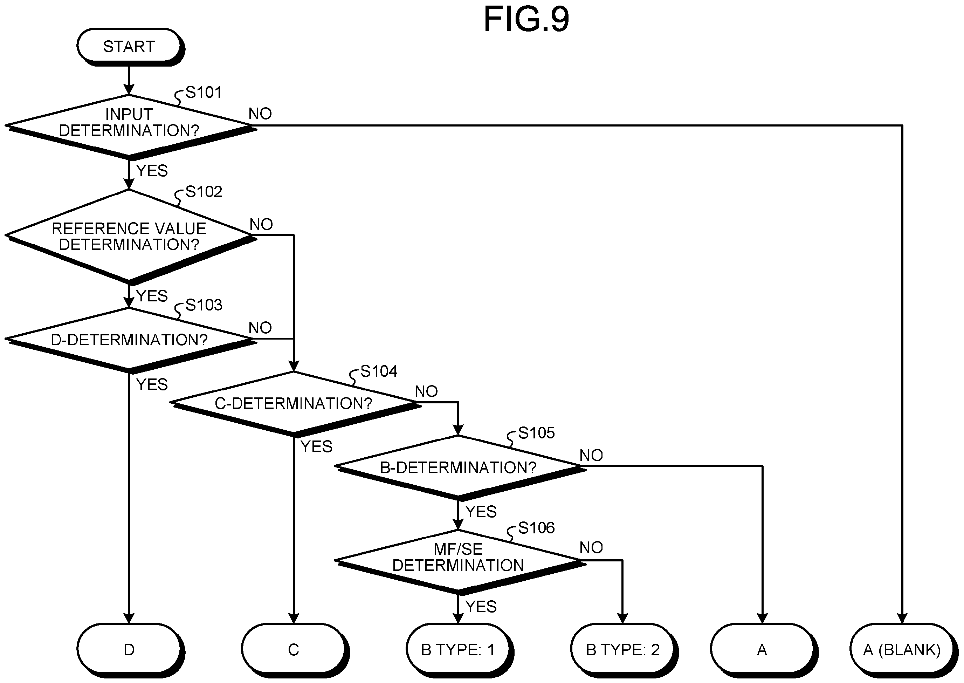

The calculation instruction unit 103 first instructs the brain function risk calculation unit 107 to calculate a brain function risk of the patient as the process of determining the brain health condition (S13). In response to the instruction, the brain function risk calculation unit 107 implements a brain function risk determination flow (for example, a determination flow in FIG. 9 or the like) by using the spectral analysis result of the patient stored in the input data storage unit 104, and calculates the brain function risk of the patient.

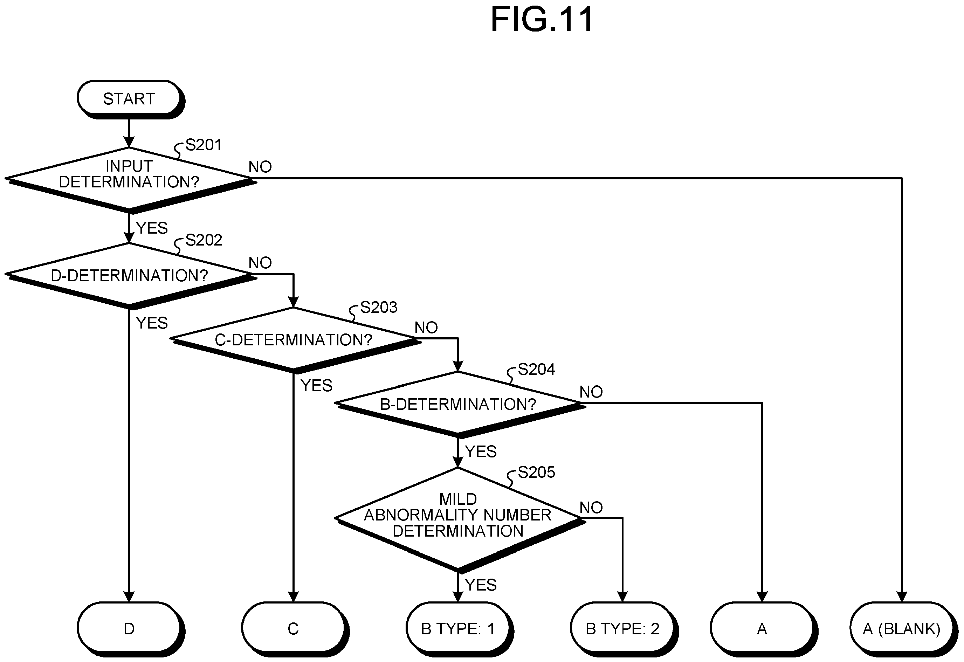

If the brain function risk calculation unit 107 completes the calculation, the calculation instruction unit 103 subsequently instructs the cognitive function risk calculation unit 108 to calculate a cognitive function risk of the patient (S14). In response to the instruction, the cognitive function risk calculation unit 108 implements a cognitive function risk determination flow (for example, a determination flow in FIG. 11 or the like) by using the psychological test result of the patient stored in the input data storage unit 104, and calculates the cognitive function risk of the patient.

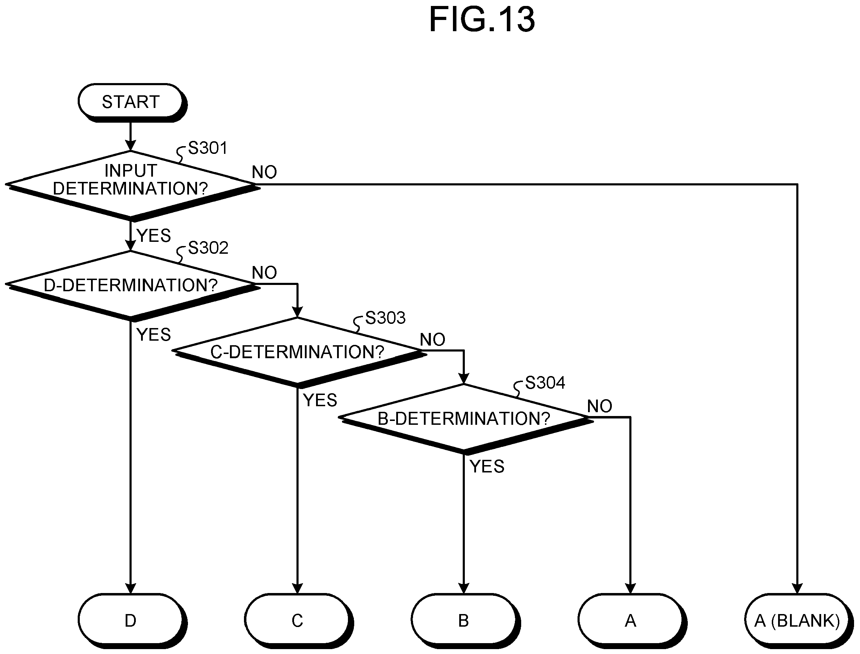

If the cognitive function risk calculation unit 108 completes the calculation, the calculation instruction unit 103 subsequently instructs the lifestyle risk calculation unit 109 to calculate a lifestyle risk of the patient (S15). In response to the instruction, the lifestyle risk calculation unit 109 performs a lifestyle risk determination flow (for example, a determination flow in FIG. 13 or the like) by using the medical interview result on the patient stored in the input data storage unit 104, and calculates the lifestyle risk of the patient.

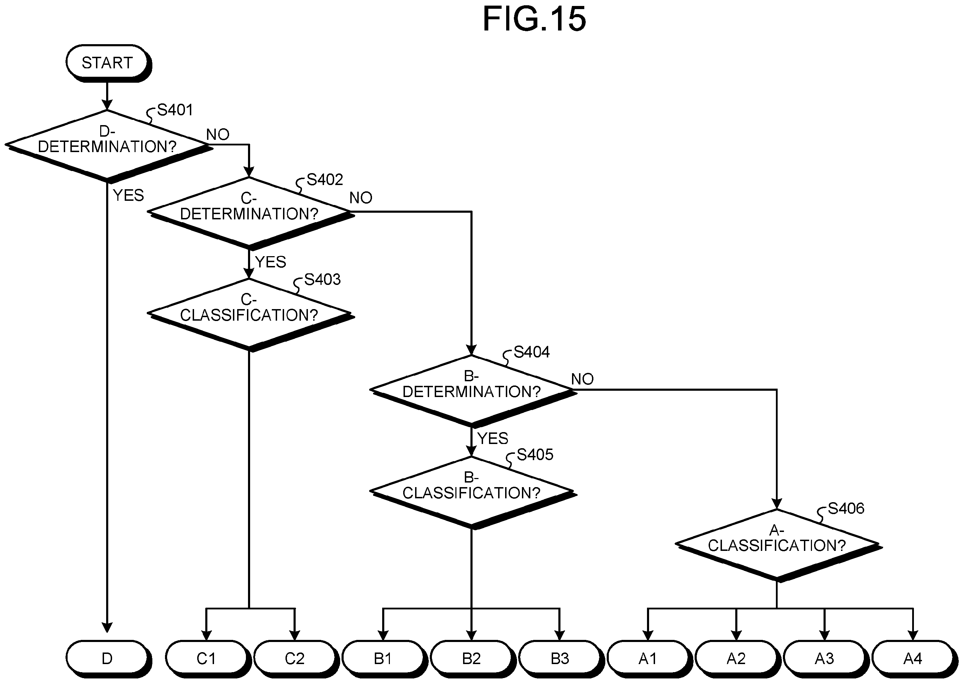

If the lifestyle risk calculation unit 109 completes the calculation, the calculation instruction unit 103 subsequently instructs the comprehensive evaluation calculation unit 110 to determine the brain health condition of the patient (S16). In response to the instruction, the comprehensive evaluation calculation unit 110 implements a comprehensive evaluation flow (for example, a determination flow in FIG. 15 or the like) by using an obtained calculation result among the results of the brain function risk, the cognitive function risk, and the lifestyle risk, and calculates the comprehensive evaluation on the brain health condition of the patient. The result calculated by each of the units is stored in the input data storage unit 104, and the interface controller 101 gives a completion notice to the client PC 20.

Subsequently, the medical examination representative operates the UI screen and requests the web server 10 (the interface controller 101) to generate a report of the brain health condition of the patient (S17).

Upon receiving the request for generation of the report of the brain health condition of the patient from the client PC 20, the interface controller 101 acquires a report of the brain health condition of the patient from the report generation unit 105 (S18). The report generation unit 105 generates a report file based on various kinds of data on the patient stored in the input data storage unit 104.

Then, the interface controller 101 transmits the report file of the patient to the client PC 20 (S19), and contents of the report indicating the brain health condition of the patient are displayed on the UI screen of the client PC 20.

Meanwhile, for example, if the psychological test is not performed, the result of the cognitive function risk is not obtained and a piece of information is missing; however, by implementing the comprehensive determination flow, an evaluation is performed using the results of the two risks on the basis of a dependence relationship between the brain function risk and the lifestyle risk, and the report file on the evaluation is transmitted to the client PC 20.

The medical examination representative outputs the report file, and provides the contents of the report to the patient in the form of an electronic file or a paper medium (S20). Thereafter, the patient connects to the web server 10 (the interface controller 101) via the mobile terminal device 30 from home or a different facility by using the authentication information, such as the patient ID. For example, the connection is established by performing authentication using login information (the patient ID or the like) on the report file.

Then, in the mobile terminal device 30, the UI screen acquired from the web server 10 is operated, the psychological test D4 of the patient is newly input or contents of answers for the medical interview D3 or the psychological test D4 are input again, and the input data (the medical interview result D3 or the psychological test D4) is transmitted to the web server 10 (the interface controller 101) (S21). The interface controller 101 additionally registers the input data on the patient transmitted from the mobile terminal device 30 in the input data storage unit 104 in association with the patient ID of the patient (S22) .

If additional registration is performed, the comprehensive determination process is performed in the same manner as the comprehensive determination (S11 to S16) (not illustrated), and the report generation unit 105 generates a report including an updated comprehensive determination result.

Thereafter, in the mobile terminal device 30, the UI screen is further operated, and a request to re-acquire the report on the brain health condition of the patient is issued to the web server 10 (the interface controller 101) (S23). In response to the request, the interface controller 101 acquires the report that is generated based on the updated data from the report generation unit 105, and transmits the report to the mobile terminal device 30 (S24). Contents of the report indicating the updated brain health condition are displayed on the UI screen of the mobile terminal device 30.

Meanwhile, it is possible to update the data registered in the web server 10 from the client PC 20 and the mobile terminal device 30 as the occasion demands. If the updated data is repeatedly transmitted from the client PC 20 or the mobile terminal device 30, the report generation unit 105 generates a history of update of the comprehensive determination at each of transmission dates and times, and outputs the history as certain information, such as a graph, which is represented in chronological order in the report.

Meanwhile, it may be possible to limit transmission information on the interface controller 101 side such that only information needed for the patient or a person who supports the patient is displayed on the UI screen of the mobile terminal device 30. For example, the UI screen of the mobile terminal device 30 limits the transmission information to only functions to register and update the input data, such as the medical interview or the psychological test, and to display the report.

### UI screen

The UI screen will be described below. Here, an example of the UI screen in which various registration and input screens are switched by tabs is illustrated.

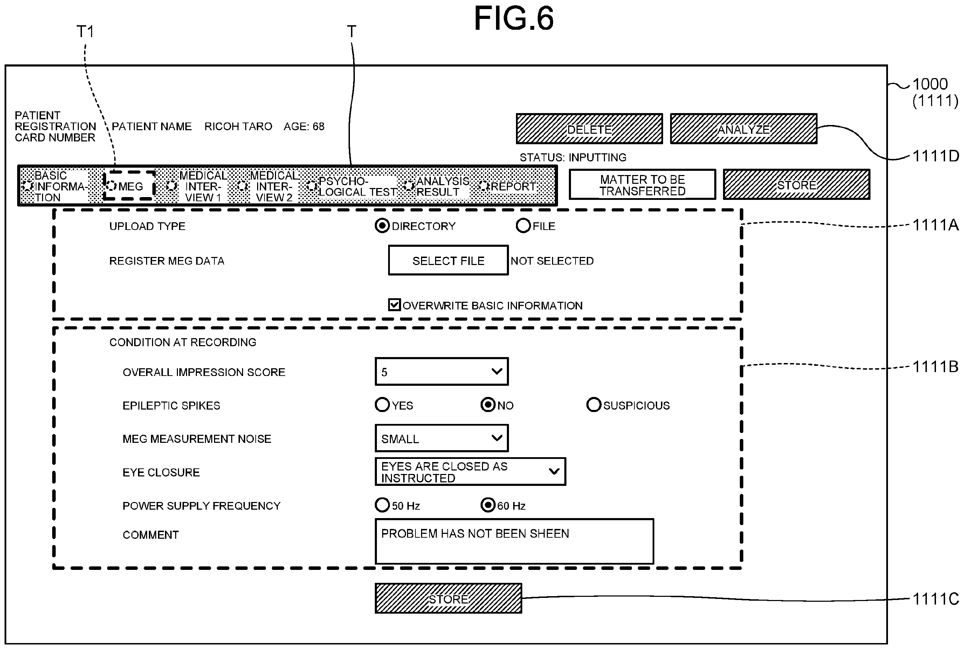

FIG. 6 is a diagram illustrating an example of a registration screen for magnetoencephalography data, which is displayed on the UI screen. In this example, a UI screen 1000 includes a plurality of switching tabs T in an upper portion thereof, and, by switching the display to an MEG tab T1 among the switching tabs T, a registration screen 1111 for the magnetoencephalography data is displayed such that input is acceptable as illustrated in FIG. 6. The plurality of switching tabs T include a reference information tab, a medical interview tab, a psychological test tab, an analysis result tab, a report tab, and the like. The reference information tab is provided on the UI screen of the client PC 20, and receives input of the patient identification information D2 indicating a name, an age, a gender, and the like of a patient from a staff or the like in a hospital or a medical examination facility.

The registration screen 1111 illustrated in FIG. 6 includes a file selection box 1111A for the magnetoencephalography data measured by the magnetoencephalograph 40 (see FIG. 1), a setting input box 1111B for adding information to be checked by the magnetoencephalography data, and the like. The registration screen 1111 is provided on the UI screen of the client PC 20. The staff or the like in the hospital or the medical examination facility selects the file of the magnetoencephalography data of the patient in the file selection box 1111A in the registration screen 1111, sets information to be checked by the magnetoencephalography data in the setting input box 1111B, and presses a storage button 1111C. If the storage button 1111C is pressed, the magnetoencephalography data on the patient is stored, as a file, from the magnetoencephalograph to the client PC 20. Then, if an analysis button 1111D is pressed, the client PC 20 transmits the file of the magnetoencephalography data D1 (including the set information) to the web server 10 (the interface controller 101).

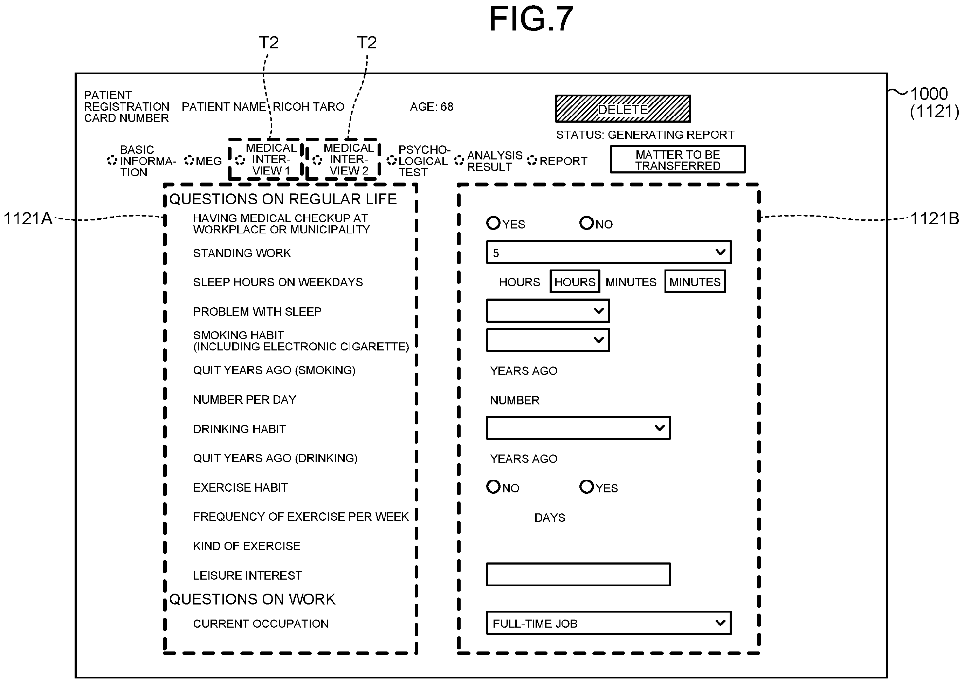

FIG. 7 is a diagram illustrating an example of an input screen for questions and answers for a medical interview. An input screen 1121 is provided to the UI screen of the mobile terminal device 30 in addition to the client PC 20, and, by switching the display to a medical interview tab T2, the input screen 1121 is displayed such that input is acceptable. One example of questions for the medical interview (an example of a medical interview 1) is illustrated in the input screen 1121 in FIG. 7. The questions include questions for recognizing a behavior pattern, a lifestyle, a personality, and the like of the patient. An answer for each of questions 1121A is input by the patient him or herself or an assistant staff for the patient by designation in a selection field 1121B, and the medical interview result D3 is transmitted to the web server 10 (the interface controller 101) by pressing a storage button. The input screen 1121 is obtained by the mobile terminal device 30 through an access to the web server 10; therefore, it is possible for the patient him or herself or the assistant staff for the patient to update the answers for the medical interview through the UI screen of the mobile terminal device 30 as the occasion demands.

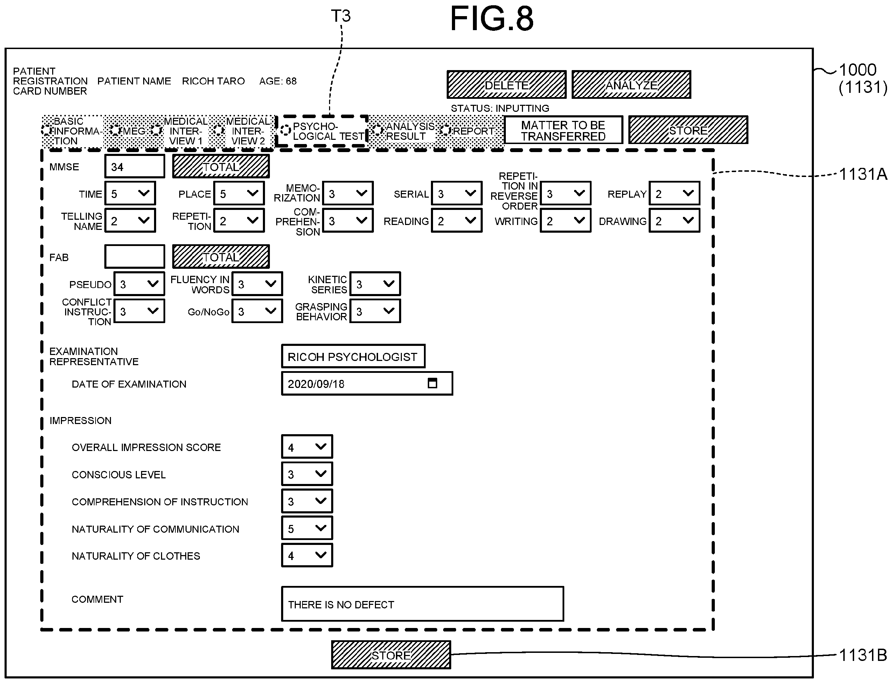

FIG. 8 is a diagram illustrating an example of an input screen for inputting a result of a psychological test. An input screen 1131 is provided to the UI screen of the mobile terminal device 30 in addition to the client PC 20 similarly to the input screen 1121 for answers for the medical interview, and, by switching the display to a psychological test tab T3, the input screen 1131 is displayed such that input is acceptable. One example of items of the psychological test is illustrated in the input screen 1131 in FIG. 8. Input items for the Mini-Mental State Examination (MMSE), the Frontal Assessment Battery (FAB), and the like are included. In each of items 1131A, a rank of an impression that is interpreted by a psychologist, the assistant staff, or the like from the patient is specified and input, and an input result (the psychological test result D4) is transmitted to the web server 10 (the interface controller 101) by pressing a storage button 1131B. Further, it is possible to obtain the input screen 1131 by accessing the web server 10 from the mobile terminal device 30; therefore, it is possible to update a result, such as the rank of the psychological test, on the UI screen of the mobile terminal device 30 as the occasion demands.

### Determination flow

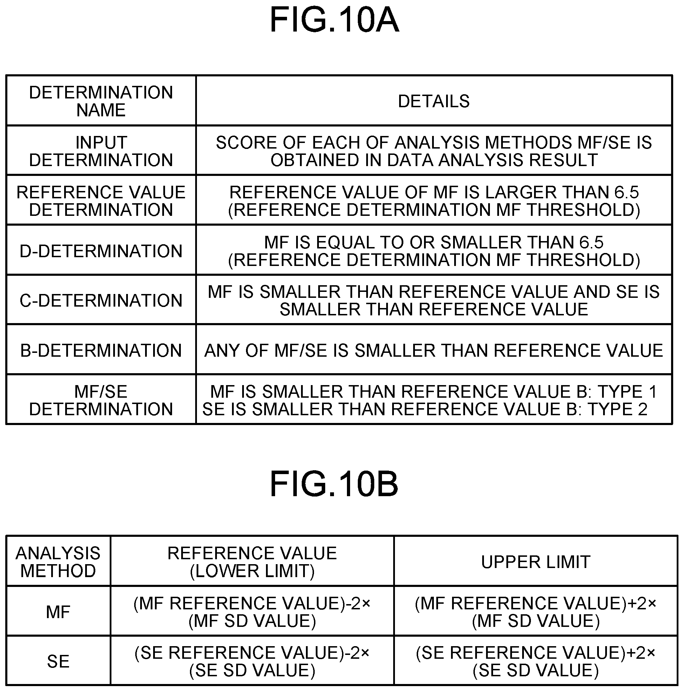

FIG. 9 is a diagram illustrating an example of a flow of determining the brain function risk by the brain function risk calculation unit 107. FIGS. 10A and 10B are diagrams illustrating an example of determination criteria for determining the brain function risk. In the flow of determining the brain function risk illustrated in FIG. 9, the brain function risk is determined based on the determination criteria illustrated in FIGS. 10A and 10B. As one example of the determination criteria, values (scores) of a brain rhythm and complexity of brainwaves that are calculated by the spectral analysis on the magnetoencephalography data. Here, the brain rhythm is mean frequency (MF) and the complexity of brainwaves is spectrum entropy (SE).

First, the brain function risk calculation unit 107 determines whether a spectral analysis result (score) of each of analysis methods MF and SF is obtained as indicated by details of input determination (see FIGS. 10A and 10B) (S101). If the spectral analysis result (score) of each of the analysis methods MF and SF is not obtained (NO at S101), the brain function risk calculation unit 107 determines that the brain function risk is "A (blank)".

If the spectral analysis result (score) of each of the analysis methods MF and SF is obtained (YES at S101), the brain function risk calculation unit 107 determines whether the MF score meets details of reference value determination (see FIGS. 10A and 10B) (S102).

If the MF score meets the details of the reference value determination (see FIGS. 10A and 10B) (YES at S102), the brain function risk calculation unit 107 determines whether the MF score further meets details of D-determination (see FIGS. 10A and 10B) (S103). If the MF score meets the details of the D-determination (see FIGS. 10A and 10B) (YES at S103), the brain function risk calculation unit 107 determines that the brain function risk is "D".

If the MF score does not meet the details of the reference value determination (see FIGS. 10A and 10B) (NO at S102), or if the MF score does not meet the details of the D-determination (see FIGS. 10A and 10B) (NO at S103), the brain function risk calculation unit 107 determines whether the MF score and the SE score meet details of C-determination (see FIGS. 10A and 10B) (S104). If the MF score and the SE score meet the details of the C-determination (see FIGS. 10A and 10B) (YES at S104), the brain function risk calculation unit 107 determines that the brain function risk is "C".

If the MF score and the SE score do not meet the details of the C-determination (NO at S104), the brain function risk calculation unit 107 determines whether any of the MF and the SE meets details of B-determination (see FIGS. 10A and 10B) (S105).

If any of the MF and the SE meets the details of the B-determination (see FIGS. 10A and 10B) (YES at S105), the brain function risk calculation unit 107 further performs MF/SE determination (S106).

If the MF meets details of the MF/SE determination (see FIGS. 10A and 10B) (YES at S106), the brain function risk calculation unit 107 determines that the brain function risk is "B type: 1". If the SE meets the details of the MF/SE determination (see FIGS. 10A and 10B) (NO at S106), the brain function risk calculation unit 107 determines that the brain function risk is "B type: 2".

If both of the MF and the SE do not meet the details of the B-determination (see FIGS. 10A and 10B) (NO at S105), the brain function risk calculation unit 107 determines that the brain function risk is "A".

In this manner, even if the magnetoencephalography data is not obtained, the brain function risk is determined as "A" because of the comprehensive determination. However, because the determination on the brain function is temporary determination, an identifier indicating blank is added.

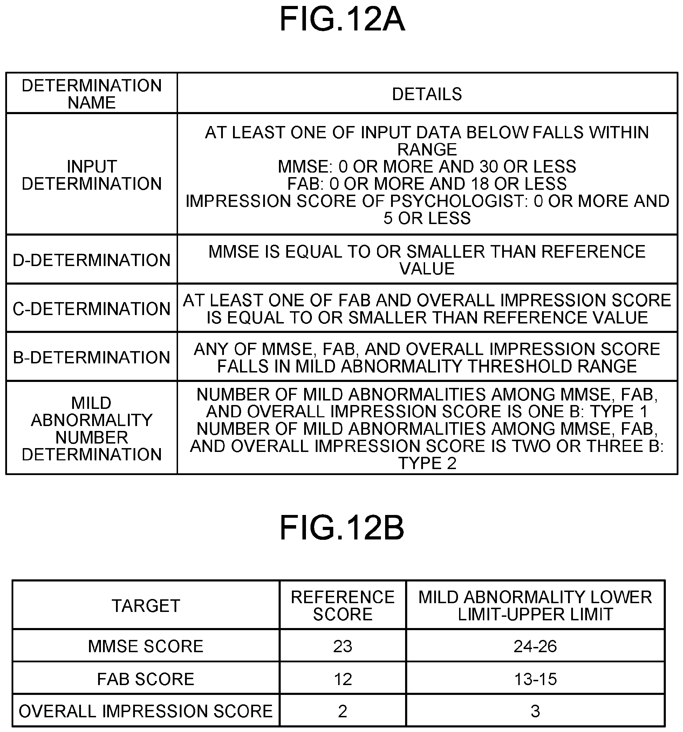

FIG. 11 is a diagram illustrating an example of a flow of determining the cognitive function risk by the cognitive function risk calculation unit 108. FIGS. 12A and 12B are diagrams illustrating an example of determination criteria for determining the cognitive function risk. Two kinds of examinations, i.e., the MMSE and the FAB, are performed as the psychological test, and each of scores is compared with determination criteria of reference data.

First, the cognitive function risk calculation unit 108 performs input determination on input data (S201), and if the input data does not meet the input determination (see FIGS. 12A and 12B) (NO at S201), the cognitive function risk calculation unit 108 determines that the cognitive function risk is "A (blank) ".

If the input data meets the input determination (see FIGS. 12A and 12B) (YES at S201), the cognitive function risk calculation unit 108 determines whether the MMSE meets details of D-determination (see FIGS. 12A and 12B) (S202). If the MMSE meets the details of the D-determination (YES at S202), the cognitive function risk calculation unit 108 determines that the cognitive function risk is "D".

If the MMSE does not meet the details of the D-determination (see FIGS. 12A and 12B) (NO at S202), the cognitive function risk calculation unit 108 determines whether the FAB or an overall impression score meets details of C-determination (see FIGS. 12A and 12B) (S203). If the FAB or the overall impression score meets the details of the C-determination (YES at S203), the cognitive function risk calculation unit 108 determines that the cognitive function risk is "C".

If both of the FAB and the overall impression score do not meet the details of the C-determination (see FIGS. 12A and 12B) (NO at S203), the cognitive function risk calculation unit 108 determines whether the MMSE, the FAB, or the overall impression score meets details of B-determination (see FIGS. 12A and 12B) (S204).

If the MMSE, the FAB, or the overall impression score meets the details of the B-determination (YES at S204), the cognitive function risk calculation unit 108 further determines whether the number of mild abnormalities meets details of mild abnormality number determination (see FIGS. 12A and 12B) (S205). If the number meets the details of the mild abnormality number determination (YES at S205), the cognitive function risk calculation unit 108 determines that the cognitive function risk is "B type: 1". In contrast, if the number does not meet the details of the mild abnormality number determination (NO at S205), the cognitive function risk calculation unit 108 determines that the cognitive function risk is "B type: 2".

Further, if the MMSE, the FAB, or the overall impression score does not meet the details of the B-determination (NO at S204), the cognitive function risk calculation unit 108 determines that the cognitive function risk is "A".

In this manner, even if the psychological test is not obtained, the cognitive function risk is determined as "A" because of the comprehensive determination. However, because the determination on the cognitive function is temporary determination, an identifier indicating blank is added.

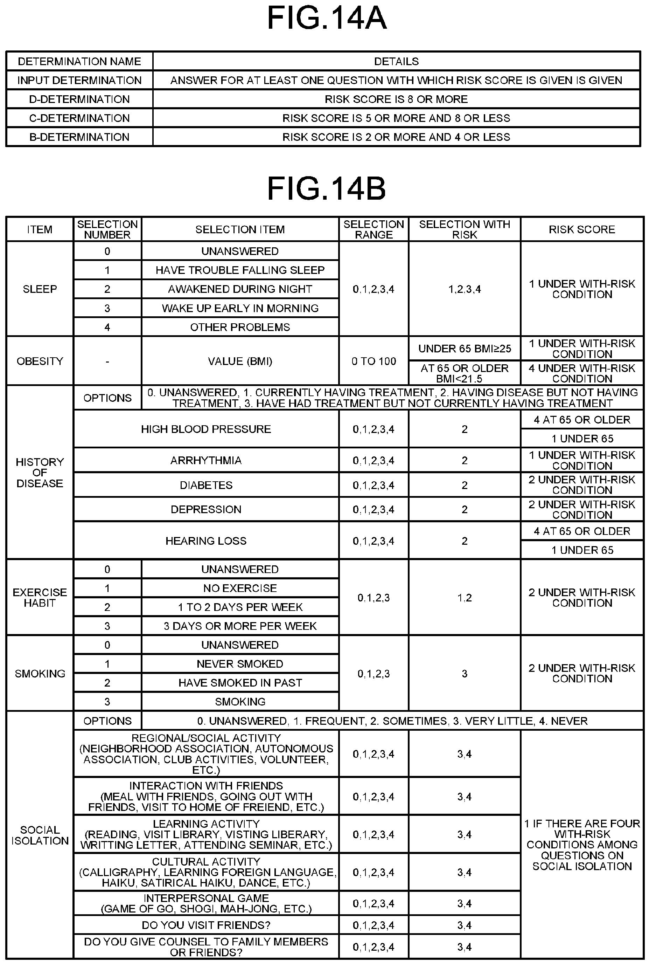

FIG. 13 is a diagram illustrating an example of a flow of determining the lifestyle risk by the lifestyle risk calculation unit 109. FIGS. 14A and 14B are diagrams illustrating an example of determination criteria for determining the lifestyle risk. The lifestyle risk is determined based on a risk score illustrated in FIG. 14A. The risk score is a score that is obtained by addition based on conditions that are determined based on a result of the medical interview. FIG. 14B illustrates an example of addition conditions for the risk score. In FIG. 14B), as one example, a score of 1 or more is given if the number of an answer for a question matches the condition. In the following, the flow of determining the lifestyle risk will be described in detail based on the assumption that the lifestyle risk calculation unit 109 has already calculated the risk score from the medical interview result.

First, the lifestyle risk calculation unit 109 performs input determination on questions and answers for the medical interview (S301), and if the questions and answers do not meet the input determination (see FIGS. 14A and 14B) (NO at S301), the lifestyle risk calculation unit 109 determines that the lifestyle risk is "A (blank)".

If the questions and answers meet the input determination (see FIGS. 14A and 14B) (YES at S301), the lifestyle risk calculation unit 109 determines whether the risk score for the questions and answers meets details of D-determination (see FIGS. 14A and 14B) (S302). If the risk score for the questions and answers meets the details of the D-determination (YES at S302), the lifestyle risk calculation unit 109 determines that the lifestyle risk is "D".

If the risk score for the questions and answers does not meet the details of the D-determination (see FIGS. 14A and 14B) (NO at S302), the lifestyle risk calculation unit 109 determines whether the risk score for the questions and answers meets details of C-determination (see FIGS. 14A and 14B) (S303). If the risk score for the questions and answers meets the details of the C-determination (YES at S303), the lifestyle risk calculation unit 109 determines that the lifestyle risk is "C".

If the risk score for the questions and answers does not meet the details of the C-determination (see FIGS. 14A and 14B) (NO at S303), the lifestyle risk calculation unit 109 determines whether the risk score for the questions and answers meets details of B-determination (see FIGS. 14A and 14B) (S304). If the risk score for the questions and answers meets the details of the B-determination (YES at S304), the lifestyle risk calculation unit 109 determines that the lifestyle risk is "B".

If the risk score for the questions and answers does not meet the details of the B-determination (see FIGS. 14A and 14B) (NO at S304), the lifestyle risk calculation unit 109 determines that the lifestyle risk is "A".

In this manner, even if the questions and answers for the medical interview are not fully obtained, the lifestyle risk is determined as "A" because of the comprehensive determination. However, because the determination on the lifestyle is temporary determination, an identifier indicating blank is added.

FIG. 15 is a diagram illustrating an example of the comprehensive evaluation flow implemented by the comprehensive evaluation calculation unit 110. The comprehensive evaluation calculation unit 110 performs comprehensive determination by using the determination results obtained through the flows illustrated in FIG. 9, FIG. 11, and FIG. 13, that is, the determination results of the brain function risk, the cognitive function risk, and the lifestyle risk (hereinafter, a combination will be referred to as a "determination result set") through the comprehensive determination flow in FIG. 15 in which a dependence relationship among the determination results is defined.

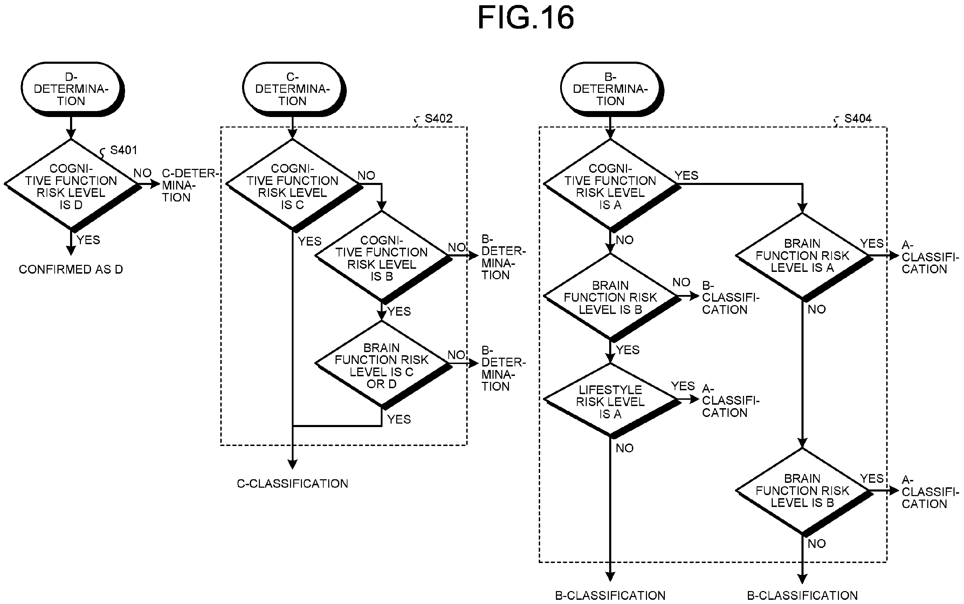

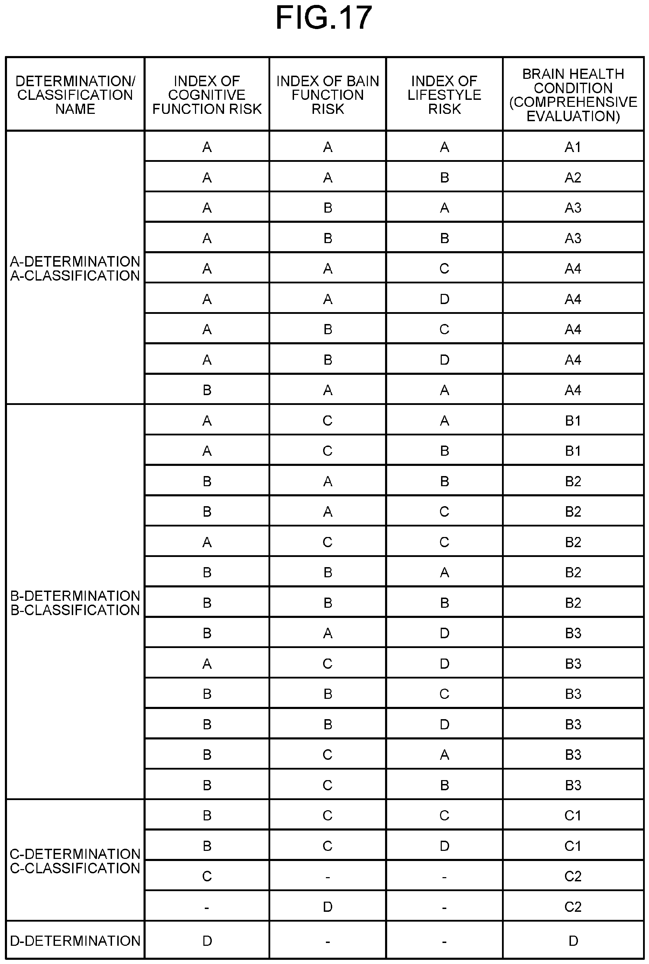

FIG. 16 is a diagram illustrating an example of a flow of a subroutine of the comprehensive evaluation flow. FIG. 17 is a diagram illustrating an example of determination criteria used for risk classification to perform a comprehensive evaluation.

The comprehensive determination flow in FIG. 15 and FIG. 16 will be described in detail below. First, the comprehensive evaluation calculation unit 110 determines whether the determination result set meets D-determination, that is, performs a D-determination process in FIG. 16 (S401), and if the determination result set meets the D-determination (YES at S401), the comprehensive evaluation calculation unit 110 determines that the comprehensive determination is "D". In the example of the D-determination process in FIG. 16, it is determined as "Yes" if the cognitive function risk is "D", and it is determined as "No" in other cases.

If the determination result set does not meet the D-determination (NO at S401), the comprehensive evaluation calculation unit 110 determines whether the determination result set meets C-determination, that is, performs C-determination in FIG. 16 (S402), and if the determination result set meets conditions for the C-determination (YES at S402 in FIG. 15), the comprehensive evaluation calculation unit 110 performs determination on C-classification (S403). In the example of the C-determination process in FIG. 16, the conditions for the C-determination are met if the cognitive function risk is "C" or even if the cognitive function risk is "B" rather than "C" and the brain function risk is "C" or "D", and the conditions for the C-determination are not met in other cases.

As for the determination on the C-classification, a value (C1 or C2) that is set in a comprehensive evaluation item corresponding to the combination of the determination results as the determination result set and that is extracted from a field of C-classification in a table for risk classification (see FIG. 17) is adopted as a determination result.

If the determination result set does not meet the C-determination (NO at S402 in FIG. 15), the comprehensive evaluation calculation unit 110 determines whether the determination result set meets B-determination, that is, whether B-determination is obtained in a B-determination process in FIG. 16 (S404), and if the determination result set meets the B-determination (YES at S404 in FIG. 15), the comprehensive evaluation calculation unit 110 performs determination on B-classification (S405). In the example of the B-determination process in FIG. 16, in a case of a combination in which the cognitive function risk is other than "A" and the brain function risk is other than "B", the process proceeds to the B-classification. Alternatively, even in a case of a combination in which the cognitive function risk is other than "A", the brain function risk is "B", and the lifestyle risk is other than "A", the process proceeds to the B-classification. In a case of a combination in which the cognitive function risk is other than "A", the brain function risk is "B", and the lifestyle risk is "A", the process proceeds to A-classification (S406 in FIG. 15). Furthermore, in a case of a combination in which the cognitive function risk is "A", the brain function risk is other than "A", and the brain function risk is other than "B", the process proceeds to the B-classification (S405 in FIG. 15). Moreover, in a case of a combination in which the cognitive function risk is "A" and the brain function risk is "A", the process proceeds to the A-classification (S406 in FIG. 15). Furthermore, in a case of a combination in which the cognitive function risk is "A", the brain function risk is other than "A", and the brain function risk is "B", the process proceeds to the A-classification (S406 in FIG. 15).

As for the determination on the B-classification, a value (B1, B2, or B3) that is set in the comprehensive evaluation item corresponding to the combination of the determination results as the determination result set and that is extracted from a field of B-classification in the table for risk classification (see FIG. 17) is adopted as a determination result.

If the determination result set does not meet the B-determination (NO at S404), the comprehensive evaluation calculation unit 110 performs determination on A-classification (S406). As for the determination on the A-classification, a value (A1, A2, A3, or A4) that is set in the comprehensive evaluation item corresponding to the combination of the determination results as the determination result set and that is extracted from a field of A-classification in the table for risk classification (see FIG. 17) is adopted as a determination result.

### Report generation

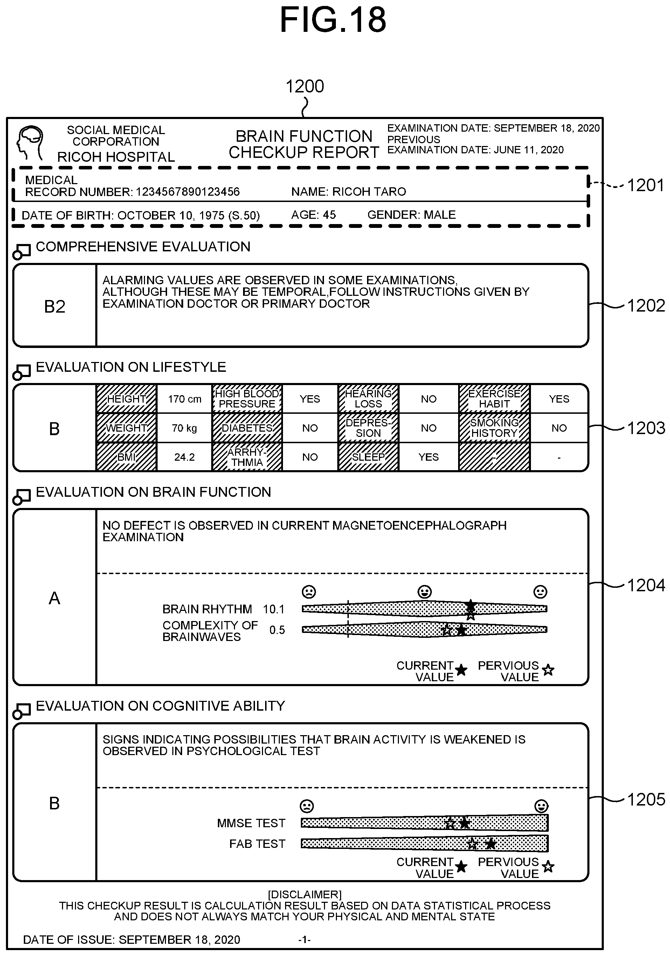

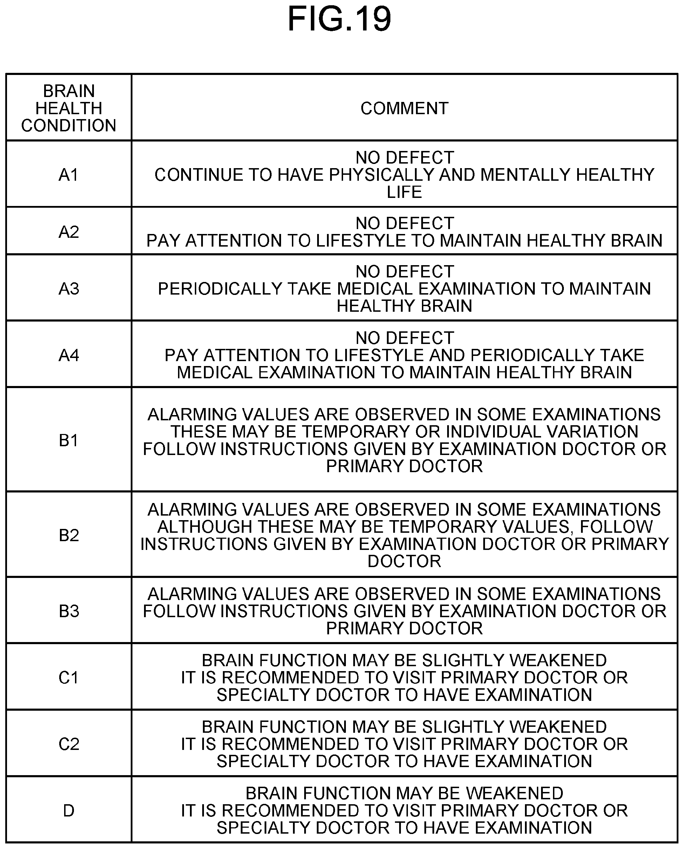

FIG. 18 is a diagram illustrating an example of a configuration of a report generated by the report generation unit 105. FIG. 19 is a diagram illustrating an example of comment data for each of categories of a comprehensive evaluation, which is used in the report.

The report illustrated in FIG. 18 is obtained by selecting a report tab among the plurality of switching tabs T illustrated in FIG. 6, for example. A report 1200 in FIG. 18 includes a patient basic information field 1201, a comprehensive evaluation field 1202, a lifestyle evaluation field 1203, a brain function evaluation field 1204, a cognitive ability evaluation field 1205, and the like. Information is set in each of the fields on the basis of the patient data stored in the input data storage unit.

For example, the comprehensive evaluation (setting of "B2" as one example) that is determined through the comprehensive evaluation determination flow and a comment for the comprehensive evaluation "B2" that is included in the commend data in FIG. 19 are set in the comprehensive evaluation field 1202.

Further, the determination result (setting of "B" as one example) that is determined through the flow of determining the lifestyle risk and the patient data related to the lifestyle are set in the lifestyle evaluation field 1203.

Furthermore, the determination result (setting of "A" as one example) that is determined through the flow of determining the the brain function risk and the level of each of the MF and the SE are set by using star marks in the brain function evaluation field 1204.

Moreover, the determination result (setting of "B" as one example) that is determined through the flow of determining the cognitive function risk and the value of each of the MMSE and the FAB are set by using star marks in the cognitive ability evaluation field 1205.

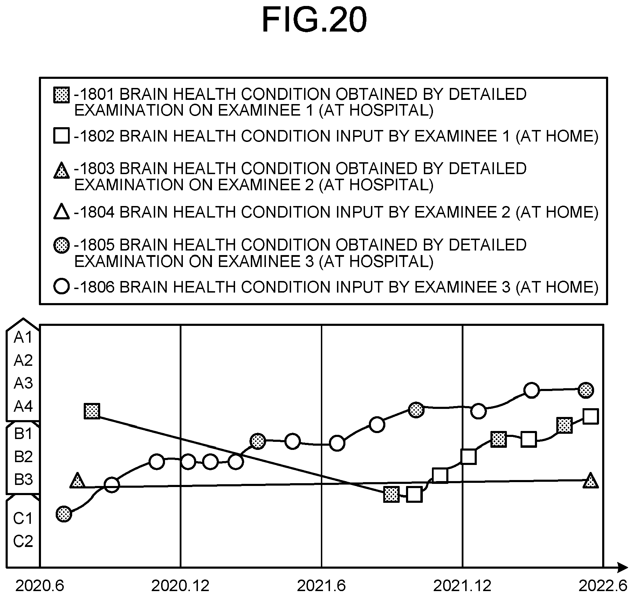

FIG. 20 is a diagram illustrating a display example in which changes in the comprehensive evaluation are displayed in chronological order on the UI screen. The comprehensive evaluation is continually changed due to update of the medical interview, the psychological test, or the magnetoencephalography data. If missing data is added, or the score of the medical interview or the psychological test is changed due to treatment, the comprehensive evaluation is changed. For example, if the score of the medical interview or the psychological test is improved due to treatment, the comprehensive evaluation is changed toward improvement.

If the medical interview, the psychological test, or the magnetoencephalography data is updated (including a case in which any of the medical interview, the psychological test, and the magnetoencephalography data is first input at a later time), the web server 10 stores a current comprehensive evaluation in addition to past comprehensive evaluations, and generates an update history. For example, a comprehensive evaluation is performed even if data is missing, and if the missing data is added, a more accurate comprehensive evaluation is stored in chronological order. Further, as illustrated in FIG. 20, a comprehensive evaluation (white plot), which is calculated every time the medical interview or the psychological test is updated at home or the like between previous visit to the hospital (colored plot) and current visit to the hospital (colored plot), is added, so that it is possible to more precisely analyze the effect of treatment performed at home.

The program executed by a computer according to the present embodiment may be provided by being incorporated in a ROM in advance. Further, the program may be provided by being recorded in a computer readable recording medium, such as a compact disk (CD)-ROM, a flexible disk (FD), a CD-recordable (CD-R), or a digital versatile disk (DVD), in a computer-installable or computer-executable file format. Furthermore, the program may be provided by being stored in a computer connected to a network, such as the Internet, and by being downloaded via the network.

As described above, according to the present embodiment, the brain health condition is determined by using at least a part (one, two, or three) of the "brain function risk", the "cognitive function risk", and the "lifestyle risk", and information for leading to improvement in the quality of life and a change in behaviors is reported, as a report on the current condition, to a doctor and a patient. If it is possible to calculate a result of at least one of the three risks, the current brain health condition is calculated using a predetermined algorithm and provided, as a report, to the doctor and the patient, so that it is possible to increase opportunities to change behaviors.

Furthermore, the dependence relationship among the risks is defined by execution of various determination flows, so that is is possible to comprehensively determine the brain function. It is possible to perform comprehensive determination by temporarily determining a risk as "A" as for any of the risks for which data is not obtained and determination is not performed among the magnetoencephalography data, the medical interview, and the psychological test. As for data that is not yet obtained, if the data is input at a later time, the remaining risk is determined and the comprehensive determination is performed again. Furthermore, if the magnetoencephalography data, the data of the medical interview, or the data of the psychological test is updated, the comprehensive determination is performed again based on the latest data.

Therefore, it is possible to comprehensively evaluate the brain function on the basis of data (examinee information) on the brain function of the examinee even from a part (for example, two risks) of the brain function risk, the cognitive function risk, and the lifestyle risk. Consequently, even if it is difficult to perform all kinds of measurement in the hospital due to the health condition of the patient, it is possible to immediately start appropriate treatment by referring to the comprehensive evaluation information.

Furthermore, it is possible to evaluate the brain function as the occasion demands, on the basis of the information on the brain function obtained from the patient. By performing the psychological test or the like as the occasion demands while having treatment at home or the like, the patient is able to confirm a result of an objective treatment effect by the brain function evaluation system. Therefore, even if the patient him or herself feels that the treatment is ineffective, the objective effect given by the brain function evaluation system may lead to an increase in motivation and encourage the patient to continue the treatment.

Moreover, if latest information obtained at a certain place, such as home or an intervention facility, with respect to a certain factor is present, it is possible to add or update the information in the past report, and it is possible to update the current "brain health condition" and provide the updated brain health condition. The examinee who has obtained the updated information is able to improve motivation to change behaviors.

Furthermore, if it is difficult to achieve the effect of the treatment at home, it is possible for the patient or the assistant staff to make a determination to visit the hospital at an earlier timing or to change the treatment with consultation with the assistant staff or the like.

Moreover, it is possible to confirm the effect of a change in behaviors and improvement in lifestyle without visiting the hospital to have an examination, so that it is possible to continuously perform improvement behaviors with reduced economical and mental loads on the examinee.

The above-described embodiments are illustrative and do not limit the present invention. Thus, numerous additional modifications and variations are possible in light of the above teachings. For example, at least one element of different illustrative and exemplary embodiments herein may be combined with each other or substituted for each other within the scope of this disclosure and appended claims. Further, features of components of the embodiments, such as the number, the position, and the shape are not limited the embodiments and thus may be preferably set. It is therefore to be understood that within the scope of the appended claims, the disclosure of the present invention may be practiced otherwise than as specifically described herein.

The method steps, processes, or operations described herein are not to be construed as necessarily requiring their performance in the particular order discussed or illustrated, unless specifically identified as an order of performance or clearly identified through the context. It is also to be understood that additional or alternative steps may be employed.

Further, any of the above-described apparatus, devices or units can be implemented as a hardware apparatus, such as a special-purpose circuit or device, or as a hardware/software combination, such as a processor executing a software program.

Further, as described above, any one of the above-described and other methods of the present invention may be embodied in the form of a computer program stored in any kind of storage medium. Examples of storage mediums include, but are not limited to, flexible disk, hard disk, optical discs, magneto-optical discs, magnetic tapes, nonvolatile memory, semiconductor memory, read-only-memory (ROM), etc.

Alternatively, any one of the above-described and other methods of the present invention may be implemented by an application specific integrated circuit (ASIC), a digital signal processor (DSP) or a field programmable gate array (FPGA), prepared by interconnecting an appropriate network of conventional component circuits or by a combination thereof with one or more conventional general purpose microprocessors or signal processors programmed accordingly.

Each of the functions of the described embodiments may be implemented by one or more processing circuits or circuitry. Processing circuitry includes a programmed processor, as a processor includes circuitry. A processing circuit also includes devices such as an application specific integrated circuit (ASIC), digital signal processor (DSP), field programmable gate array (FPGA) and conventional circuit components arranged to perform the recited functions.

## Claims

1. A brain function evaluation system comprising:
a receiving unit configured to receive examinee information on a brain function; and
a processing unit configured to calculate an evaluation result by evaluating a brain function based on the examinee information and additional examinee information.

2. The brain function evaluation system according to claim 1, wherein the processing unit is configured to output the evaluation result by evaluating the brain function based on the examinee information.

3. The brain function evaluation system according to claim 1 or 2, wherein
the examinee information is information for evaluating each of a brain function risk, a cognitive function risk, and a lifestyle risk, and
the processing unit is configured to output the evaluation result depending on an evaluation result of each of the brain function risk, the cognitive function risk, and the lifestyle risk.

4. The brain function evaluation system according to claim 2 or 3, wherein
the receiving unit is configured to receive the additional examinee information from a medical staff terminal and an examinee terminal, and
the processing unit is configured to calculate the evaluation result by re-evaluating the brain function based on the examinee information on the brain function and the additional examinee information.

5. The brain function evaluation system according to any one of claims 2 to 4, wherein the receiving unit is configured to receive update information on the examinee information, and
the processing unit is configure to calculate the evaluation result by re-evaluating the brain function based on updated information obtained by updating the examinee information on the brain function with the update information.

6. The brain function evaluation system according to any one of claims 1 to 5, wherein the processing unit is configured to output the evaluation result to one of a medical staff terminal and an examinee terminal.

7. The brain function evaluation system according to claim 6, wherein the processing unit is configured to output, as a report, an update history of the evaluation result.

8. The brain function evaluation system according to claim 7, wherein the update history includes chronological information on the evaluation result.

9. A method implemented by a computer to calculate an evaluation result of a brain function, the method comprising:
receiving examinee information on the brain function;
calculating an evaluation result by evaluating the brain function based on the examinee information;
receiving additional examinee information; and
outputting an evaluation result by evaluating the brain function based on the examinee information and the additional examinee information.

10. A non-transitory computer readable medium including programmed instructions that cause a computer to function as:
a receiving unit configured to receive examinee information on a brain function; and
a processing unit configured to calculate an evaluation result by evaluating the brain function based on the examinee information and additional examinee information.
